# EUROPEAN PATENT APPLICATION

(11) **EP 3 968 338 A1**
(43) Date of publication of application: **16.03.2022**
(21) Application number: 20802047.9
(22) Date of filing: 06.05.2020
(51) Int. Cl.: G16H 50/20, G16H 10/60, C12Q 1/04, C12Q 1/68, G01N 33/48

(54) **METHOD AND SYSTEM OF GENERATING INDIVIDUAL DIETARY RECOMMENDATIONS BASED ON ANALYSIS OF MICROBIOTA COMPOSITION**

(30) Priority: 07.05.2019 RU 2019113824
(71) Applicant: Atlas Biomed Group Limited, London E1W 1DD (GB)
(72) Inventor: TIAKHT, Aleksandr Viktorovich, Moscow, 121069 (RU); POPENKO, Anna Sergeevna, Moscow, 121069 (RU); ALEXEEV, Dmitrii Glebovich, Moscow, 121069 (RU); KLIMENKO, Natalia Sergeevna, Moscow, 121069 (RU); MUSIENKO, Sergei Vladimirovich, Moscow, 121069 (RU)
(74) Representative: Papula Oy
(86) International application number: PCT/RU2020/050090
(87) International publication number: WO 2020/226535

(57) **Abstract**

The present disclosure is generally related to microbiology. A computer-implemented method and a computer system of generating a recommendation for a user for dietary intervention. The method comprises: obtaining a set consisting of at least one proposed dietary intervention for the user and data on a composition of intestinal microbiota of the user; retrieving data sets on changes in a composition of intestinal microbiota of subjects after the proposed dietary from a database of dietary interventions; assigning each selected subject to a group with a high degree of response (responder) or a group with a low degree of response (non-responder) to the proposed dietary intervention; generating an algorithm for predicting the degree of individual response to the proposed dietary intervention based on retrieved data sets of the subjects; classifying the user as a responder or a non-responder and generating a recommendation for the user for the dietary intervention.

## Description

### FIELD OF THE INVENTION

The present disclosure is generally related to microbiology, as well as to the use of computer technology in microbiology and nutrition and is more specifically related to methods and systems for study and interpretation of data on a composition of human intestinal microbiota in order to develop recommendations for the user for the use of dietary supplements and diet.

### BACKGROUND OF THE INVENTION

The human body contains about 100 trillion bacteria, which significantly exceeds the total number of eukaryotic cells of all human tissues and organs. All these human microorganisms combined are called microbiota, and the totality of their genes is called metagenome. Most of the microorganisms are in the gastrointestinal tract, therefore, study of the gastrointestinal microbiota and interpretation of these data are a very important technical problem. Recent studies have shown that changes in the species composition of microorganisms present in the gastrointestinal tract, as well as quantitative changes in certain species of gastrointestinal microorganisms can be associated with various human diseases (Shaw et al. 2016; de la Cuesta-Zuluaga et al. 2018; Cui et al. 2017; Gevers et al. 2014). This association is caused by the symbiosis between the human body and the microbiota. The microorganisms use nutrients and dietary fibers coming from food for their reproduction. In turn, they synthesize vitamins partially entering the human body and short-chain fatty acids (SCFA), some of which have anti-inflammatory, anti-cancer effects and play a key role in the regulation of human body systems. An imbalance in the microbiota composition can cause the development of chronic inflammatory conditions. This along with other factors can lead to various serious diseases: diabetes mellitus type II, Crohn's disease, ulcerative colitis, obesity, etc.

The background art contains numerous sources of information indicating the importance of using probiotics (Isolauri et al. 2000; Kim et al. 2010; Singh et al. 2013) (live symbiotic microorganisms, which taken in adequate amounts, can have a beneficial effect on human health - both separately and in the composition of food products, e.g. fermented milk products), as well as a balanced diet rich in dietary fibers (acting as prebiotics - food ingredients not digested by humans, but contributing to the growth or increase in the activity of symbiotic microorganisms) to maintain a healthy microbiota in the human gastrointestinal tract and potentially prevent the development of symptoms of the above-mentioned diseases or improve them (Khaw and Barrett-Connor 1987; Ludwig et al. 1999; Anderson et al. 1987). However, clinical studies examining the effectiveness of probiotics and prebiotics in the prevention and treatment of these diseases have shown mixed results (Rondanelli et al. 2017; Makki et al. 2018). The studies show that not all people react equally to the same dietary supplements, and the reaction of the body depends on the initial composition of the microbiota in the gastrointestinal tract of the individual. For example, the same product can cause significant changes in the gastrointestinal microflora of some individuals and cause only small changes in others (Klimenko N. S., et al., Microbiome Responses to an Uncontrolled Short-Term Diet Intervention in the Frame of the Citizen Science Project. Nutrients. 2018 May 8; 10(5)). Thus, despite the presence of general recommendations for a balanced diet for a wide range of population, there is a need for methods of developing personalized dietary recommendations for a particular patient, taking into account the composition of his/her gastrointestinal microbiota.

### SUMMARY OF THE INVENTION

The present disclosure is focused on removal of disadvantages common to the solutions known from the background art.

The technical task or technical problem solved in the present disclosure is to implement a method and a system for determining an effectiveness of dietary interventions for an individual user, including addition of dietary fibers of a certain type or other prebiotics to the diet, consumption of certain products enriched with probiotics or biologically active supplements with probiotics, as well as addition, restriction of certain food products or exclusion them from the diet.

The technical result achieved in solving the above-mentioned technical problem is to increase the effectiveness of various dietary interventions, which is assessed as a change in the composition of an intestinal microbiota of the individual user (for example, the relative abundance of microbial taxa) and/or as an effect on the health of the user.

The above-mentioned technical result is achieved through implementing the method of generating a recommendation for the user for a dietary intervention, wherein obtaining a set consisting of at least one dietary intervention for at least one user and user's data including at least data on a composition of intestinal microbiota and data on an abundance of taxa of microorganisms present in the gastrointestinal tract on a server; retrieving data sets on changes in the abundance of microbial taxa present in the gastrointestinal tract of subjects after at least one dietary intervention proposed in the previous stage by means of a processor from at least one database of dietary interventions, while selecting data sets of subjects residing in the same territory as the user; assigning each subject selected in the previous stage to the group with a high degree of response (responder) to the proposed dietary intervention or to the group with a low degree of response (non-responder) with using the value of changes in the abundance of microbial taxa present in the gastrointestinal tract after the proposed dietary intervention as a measure of the degree of response; generating an algorithm for predicting the degree of individual response to the proposed dietary intervention based on the composition of the intestinal microbiota, which classifies a user as a responder or a non-responder, from the earlier obtained data on the composition of the intestinal microbiota and the group of response to the proposed dietary intervention; classifying at least one user as a responder or a non-responder to the proposed dietary intervention based on the algorithm for predicting the degree of individual response, and generating a recommendation for the proposed dietary intervention if the user has been classified as a responder to it.

In some embodiments of the invention, the degree of response to the proposed dietary intervention is assessed additionally based on biochemical tests and/or questionnaires on gastrointestinal symptoms and/or defecation frequency and/or well-being.

In some embodiments of the invention, a biochemical test is an analysis of the level of triglycerides and other indicators in the blood and/or an analysis of faeces and/or a microbiological analysis of faeces.

In some embodiments of the invention, a random forest algorithm, a support vector machine, an artificial neural network is used as an algorithm for predicting the degree of individual response.

In some embodiments of the invention, subjects are assigned to the group of responders to the dietary intervention, for whom the value of changes in the abundance of microbial taxa after the proposed dietary intervention exceeded a threshold value, or to the group of non-responders for whom this value did not exceed the threshold value.

In some embodiments of the invention, data sets from users and/or subjects are obtained by analyzing microbiota samples obtained from the users/subjects and containing microorganisms present in their gastrointestinal tract.

In some embodiments of the invention, the obtained microbiota samples are samples of faeces.

In some embodiments of the invention, data sets from users and/or subjects are obtained by sequencing libraries prepared from DNAs extracted from the microbiota samples with the use of enzymatic lysis and/or mechanical lysis and/or washout.

In some embodiments of the invention, the standardized technique is DNA sequencing the whole or partial sequence of 16S rRNA genes or metagenomic whole genome sequencing.

In some embodiments of the invention, values of changes in the abundance of microbial taxa are calculated using preset taxa.

In some embodiments of the invention, the proposed dietary intervention is a long-term administration of a product or a variety of products rich in a certain class of dietary fibers, prebiotics, probiotics or a food product enriched with prebiotics or probiotics.

In some embodiments of the invention, the proposed dietary intervention is fasting therapy.

In some embodiments of the invention, the recommendation is to exclude a certain food product, probiotic or prebiotic from the diet based on that the user has been assigned to the group of non-responders to proposed dietary intervention.

In some embodiments of the invention, the recommendation is generated in reference to several proposed dietary interventions.

### BRIEF DESCRIPTION OF THE DRAWINGS

The features and advantages of the present disclosure will be evident from the following detailed description and attached drawings where:
Fig. 1 shows an embodiment of the method of generating personalized recommendations for diet based on an analysis of a microbiota composition as a flow chart.
Fig. 2 shows the ROC curve for the classifier enabling determination of the degree of response to a diet rich in dietary fibers based on the microbiota composition before the intervention.
Fig. 3 shows the ROC curve for the classifier enabling determination of the degree of response to the administration of a product enriched with probiotics based on the microbiota composition before the intervention.
Fig. 4 shows an embodiment of a system of generating personalized recommendations for diet based on an analysis of a microbiota composition as a flow chart.

### DETAILED DESCRIPTION OF THE INVENTION

The terms and their definitions used in the description of the present disclosure will be considered in detail below.

In this invention, a system means a computer system, an electronic computing machine (ECM), numeric control (NC), a programmable logic controller (PLC), computer-assisted command systems and any other devices capable of executing a specified, clearly determined sequence of operations (actions, commands).

A command-processing device means an electronic unit or an integrated circuit (microprocessor) which executes computer-based commands (programs). The command-processing device reads and executes computer-based commands (programs) from one or more data storage devices. A data storage device can be hard disk drives (HDD), flash memory, read-only memory (ROM), solid-state drives (SSD), optical disc drives (ODD), etc.

A program is a sequence of commands to be executed by a computer control device or a command-processing device.

The human microbiota (normal microflora, normal flora, microbiome) is the totality of all microorganisms in the human body.

DNA sequencing is determining the sequence of nucleotides in a DNA molecule. This can be understood as both amplicon sequencing (reading sequences of extracted DNA fragments from PCR reactions, such as the 16S rRNA gene or its fragments) and whole genome sequencing (reading sequences of the whole DNA present in the sample).

Reads are data representing nucleotide sequences of DNA fragments recovered with the use of a DNA sequencer.

FASTA is a format for recording DNA sequences.

FASTQ is a format for recording DNA sequences, wherein the hardware read quality of each position is recorded.

Read mapping is a bioinformatic method of analyzing the results of next-generation sequencing, which consists in determining positions in the reference base of genomes or genes, from where each specific short read could be obtained with high and highest probability.

DNA sequencing results in generating a set of reads. The length of a read in modern sequencers ranges from several hundred to several thousand nucleotides.

A biomarker is a measurable biological factor (for example, a relative abundance of a bacterial species or a bacterial genus) associated with the presence or absence of this or that condition in the host organism, for example, a certain disease.

Taxonomy is the doctrine of the principles and practice of classification and systematization of hierarchically-correlated complex entities.

A donor is a person who provides a sample of his/her microbiota for subsequent analysis of its composition or other purposes - for example, transplantation to a recipient.

Dietary intervention can be a long-term administration of prebiotics, probiotics, products rich in dietary fibers or food products enriched with probiotics.

In the description of this invention, the terms "includes" and "including" are interpreted as "includes, but not be limited to". These terms are not intended to be interpreted as "consists only of". Unless defined separately, technical and scientific terms in this description have the standard meanings common in the scientific and technical literature.

In this description of the invention, the abundance of microbial taxa means a set of quantitative data on microorganisms present in the gastrointestinal tract of subjects. Generally, these are data at the level of genera of microorganisms, collected in tables of abundance. To determine the taxonomic composition of the microbiota, taxon-specific real-time PCR or 16S rRNA gene sequencing or metagenomic whole genome sequencing (WGS) or hybridization panels of intestinal microbes are used.

The species composition of a microbiota sample is a set of values of the relative abundance of all microbial taxa detected in the sample.

α-diversity is a numerical value that characterizes the diversity of the microbial community for a single sample of the microbiota. α-diversity is calculated from data on the species composition of the microbiota using this or that algorithm.

β-diversity is a numerical value that characterizes the measure of difference between two microbial communities. This diversity between communities is an indicator of the degree of differentiation of species distribution. A possible way to find out β-diversity is to compare the species composition of different communities. The fewer common species between the two communities, the greater the β-diversity.

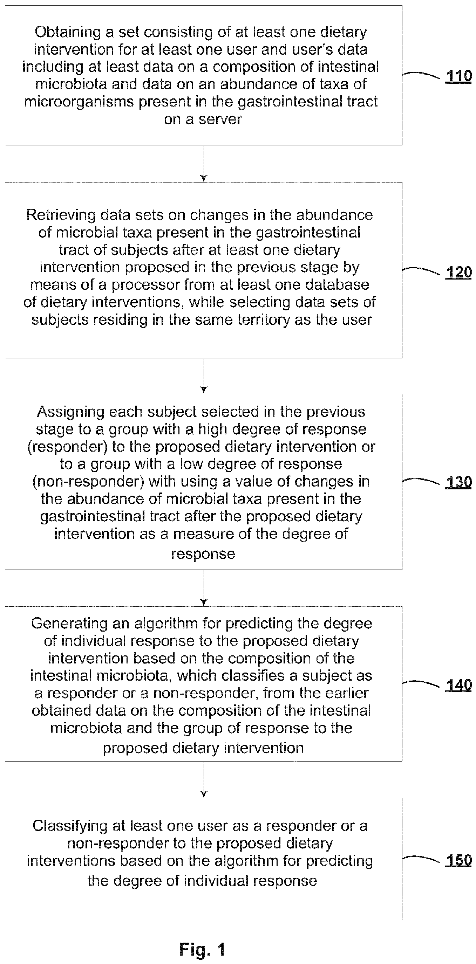

The method of generating personalized recommendations for diet based on the analysis of a microbiota composition is closely described below and is shown as a flow chart in Fig. 1.

**Step 110:** obtaining a set consisting of at least one dietary intervention for at least one user and his/her data including at least data on a composition of his/her intestinal microbiota and data on an abundance of taxa of microorganisms present in the gastrointestinal tract on a server.

In the present disclosure and in the background art, dietary intervention is understood as an increased consumption of products high in fiber, addition of dietary fibers of a certain type or other prebiotics to a diet, consumption of certain fermented milk products or other probiotics, as well as addition, restriction of certain food products or exclusion them from the diet. Concerning prebiotics, these are substances that are not absorbed or not fully absorbed by the human body, but are catabolized by intestinal microbes. Among the most common types of prebiotics are polysaccharides (such as pectin, long-chain inulin, resistant starch) and oligosaccharides (fructooligosaccharides, galactooligosaccharides, etc.). During dietary intervention, probiotics can be consumed as isolated components (in capsules) or as part of plant extracts (e.g. wheat bran). Alternatively, prebiotic intervention can be carried out by increasing the intake of products rich in this fiber (for example, topinambur and chicory in the case of inulin). When processing prebiotics with intestinal microbes, substances that have a beneficial effect on the human body - such as short-chain fatty acids (SCFA) - are secreted. Importantly, the ability to process particular types of dietary fibers varies significantly among microbial taxa, and the breakdown of some complex branched polysaccharides occurs in several stages, with one of several cooperating microbial species active in each stage. Thus, the ability of an individual intestinal community to break down different types of dietary fibers varies widely, as confirmed by a number of studies (e.g. Korpela et al. 2018, Koropatkin et al. 2012, Kaoutari et al. 2013). This creates the basis for personalizing the administration of prebiotics.

Another type of intervention is administration of probiotics - in an isolated form or as part of food products (usually, fermented milk products). Probiotic potential is a strain-specific property. Bifidobacterium (species B. animalis, B. longum, B. bifidum) and Lactobacillus (species L. rhamnosus, L. casei, L. reuteri) belong to the genera that include the largest number of used strains with probiotic properties.

Bifidobacterium animalis subsp. lactis BB-12 is often mentioned as an example of the most studied strains: it can withstand the aggressive action of bile and gastric juice in the human gastrointestinal tract and can attach to the intestinal wall; its administration may reduce the risk of acute diarrhea in children, reduce the frequency of respiratory diseases and normalize the intestinal activity (including the frequency of defecation and the consistency of faeces). Another example is Lactobacillus casei, strain Shirota: in addition to the increased ability to survive in the gastrointestinal tract, it is active against pathogenic bacterial species, normalizes the digestion and is able to modulate the immune system.

In this invention, a relative abundance of microbial taxa present in a gastrointestinal tract of subjects was studied before and after two types of dietary interventions: a diet with a high content of fibers and administration of yogurt enriched with a probiotic. For this purpose, samples of faeces of these subjects were analysed for the 16S rRNA gene. It was established that the degree of response (changes in the composition) of the gastrointestinal microbiota to the dietary interventions differed among individuals and depended on the composition of the intestinal community before the intervention.

In some embodiments of the invention, a set of data on the abundance of microbial taxa (microbiota composition) is obtained as follows.

The above-mentioned set of data is obtained using a collection kit which may include a sample container having a process reagent component and configured to receive a sample from a point of collection by a user, which can be remote. Additionally or alternatively, the collection kit can be provided directly through a device which is installed indoors or outdoors and is designated to facilitate receiving a sample from the user. In other embodiments, the collection kit can be submitted to a medical laboratory technician in a clinic or other medical institution, and earlier submitted to the user, for example, by courier. However, one or more user collection kits can be additionally or alternatively submitted to the unit of obtaining primary data by any other suitable way, for example, in frozen form in a sterile container.

Input samples can be represented by faeces samples which can be processed, for example, in a laboratory and from which data on the composition of the intestinal microbiota are obtained by means of sequencing. The processing includes the stages of sample purification from debris by means of centrifugation, total DNA extraction, including that of bacteria and archaea. Alternatively, the 16S rRNA gene or any other marker gene from the total DNA can be amplified depending on the sequencing format.

In the particular embodiment of the present disclosure, subjects (or, in other words, patients, users, etc., without limitation in terminology) involved in the study provided their microbiota samples immediately before and after the dietary intervention. The dietary intervention lasted for a certain period of time, for example, for two weeks or one month. During this period, the subjects followed the recommendations for changing the dietary plan, which depended on the planned dietary intervention. The microbiota samples were collected by the subjects on their own, frozen and delivered to the laboratory where DNA was extracted and the 16S region was sequenced (as described in Klimenko N. S., et al., Nutrients. 2018 May 8;10(5)). Sequencing was performed using the MiSeq device (Illumina, USA). The sequencing results were processed using the QIIME software v. 1.9.1 [Caporaso J. G.; et al., QIIME allows analysis of high-throughput community sequencing data. Nature methods 2010, 7, 335]. The taxonomic composition of the community was determined by aligning the sequences against HITdb v.1.0 [Ritari J.; et al., Improved taxonomic assignment of human intestinal 16S rRNA sequences by a dedicated reference database. BMC genomics 2015, 16, 1056]. In aligning, the closed-reference approach implemented using the vsearch algorithm was applied [Rognes T., Flouri T., Nichols B., Quince C., Mahe F. (2016). VSEARCH: a versatile open source tool for metagenomics. PeerJ, 4, e2584].

**Step 120:** retrieving data sets on changes in the abundance of microbial taxa present in the gastrointestinal tract of users by means of a processor from at least one database of dietary interventions after at least one proposed dietary intervention in the previous stage, while selecting only data sets of users residing in the same territory as said user.

In the particular embodiment, the metabolic potential of bacteria was further predicted using the Greengenes database v.13.5 [DeSantis T. Z.; et al., Greengenes, a chimera-checked 16S rRNA gene database and workbench compatible with ARB. Applied and environmental microbiology 2006, 72, 5069-5072] and the PICRUSt software [Langille M. G.; et al., Predictive functional profiling of microbial communities using 16S rRNA marker gene sequences. Nature biotechnology 2013, 31, 814] by means of a processor. Prediction of the metabolic potential of bacteria allows assessing the relative abundance of genes, metabolic pathways and modules in the bacterial community. In other embodiments, the metabolic potential of bacteria can be predicted using any other data processing device.

The database can be a relational database, an object-oriented database, a hierarchical database, a network database, other types of databases, a combination or an extension thereof. Data, which are a set of proposed dietary interventions, as well as user data, can be organized in the form of tables, records, objects, other data structures, etc. Data can also be stored in special database files, special partitions of hard drives, HTML files, XML files, spreadsheets, flat files, document files, configuration files, other files, etc. in the memory of a computing system **400** described below. The database can refer to a read-only set of data or can read a set of data and write to it.

When describing the aspects of this invention, sometimes terminology associated with relational databases is used herein for the sake of simplicity. However, the disclosed embodiments can be applied to other types of databases, including the above-mentioned.

**Step 130:** assigning each user of those who have data sets and have been selected in the previous stage to a group with a high degree of response (responder) to the proposed dietary intervention or to a group with a low degree of response (non-responder) with using a value of changes in the abundance of microbial taxa present in the gastrointestinal tract after the dietary intervention as a measure of the degree of response.

In some embodiments of the present disclosure, the subjects involved in the study were divided into groups depending on the degree of change in their microbiota as follows. The degree of response (changes in the microbiota composition) for each subject was calculated by calculating the β-diversity between the samples before and after the intervention using the Generalized Unifrac metric [Chen J.; et al., Associating microbiome composition with environmental covariates using generalized UniFrac distances. Bioinformatics 2012, 28, 2106-2113]. Then the users were divided into two clusters (responders and non-responders) by applying the k-means algorithm to the array of distances.

In other embodiments of the invention, both qualitative and quantitative metrics of the β-diversity between microbiota samples before and after dietary interventions can be used as a response measure. Among the quantitative metrics, Bray-Curtis, Weighted Unifrac and Generalized Unifrac distances can be used without limitation. The quality metrics used in the microbiota analysis include the Jaccard index, Unweighted Unifrac.

In other embodiments of the invention, the value of changes in the presence of bacteria associated with human health benefits, including the relative abundance of Bifidobacterium and/or Lactobacillus, lactose-fermenting bacteria in general, butyrate-producing bacteria, can be used as a response measure. The response can also be assessed as changes in the α-diversity (community diversity) as well as the level of the predicted metabolic potential of the microbiome to produce SCFA (including butyric acid).

In some embodiments of the present disclosure, bacteria differing in the relative abundance in the groups with high and low degrees of response to the dietary intervention before starting the diet were determined using the metagenomeSeq algorithm [Paulson J. N.; Pop M.; Bravo H. metagenomeSeq: Statistical analysis for sparse high-throughput sequencing, 2013. Bioconductor package: 1.16.0.].

In other embodiments of the present disclosure, bacteria differing in the relative abundance in the groups with high and low degrees of response to the dietary intervention before starting a diet can be determined using methods such as linear regression, MaAsLin algorithm [Morgan XC, et al. Dysfunction of the intestinal microbiome in inflammatory bowel disease and treatment. Genome Biol. 2012 Apr 16;13(9):R79.], generalized linear regression or Mann-Whitney criterion, but not limited to the above-mentioned.

**Step 140:** generating an algorithm for predicting the degree of individual response to the proposed intervention based on the composition of the intestinal microbiota, which classifies a user as a responder or a non-responder, from the earlier obtained data on the composition of the intestinal microbiota and the user's group of response to the proposed dietary intervention.

In some embodiments of the present disclosure, the degree of response to the dietary intervention based on the microbiota composition was predicted as follows. To determine the microbiota potential, a classifier was constructed for each study in order to predict the degree of response to the dietary intervention. To construct the classifier, the random forest algorithm was selected. The relative abundance of bacteria significantly differing in the relative abundance in the microbiota in the groups with high and low degrees of response before the diet was used as predictors. The classifier was constructed and tested at each taxonomic level.

In other embodiments of the present disclosure, linear regression, logistic regression, generalized linear models, regression with regularization, a support vector machine, a Bayesian classifier, neural networks can be used as a machine learning algorithm. In each of these methods, abundances of bacteria, metabolic pathways, modules, α-diversity, abundances of different bacterial groups can be predictors. The predictors can be transformed using mathematical methods before transferring them to the classifier, in order to better match their distribution with the selected model. If the classifier returns a numerical value corresponding to the probability that an individual is assigned to one of the clusters, a threshold value is selected, starting from which an individual will be assigned to the group with a high degree of response.

The classifier is trained on an array of data for which it is known to which group the users belong - with high or low response to a certain intervention. The sample batch should be balanced. Then this sample batch is partitioned into training and test sample sets in the ratio of 2:1. Depending on the selected machine learning method, various parameters of the algorithm are optimized for optimal classification of the training sample set. Then, the classifier characteristics (such as sensitivity, specificity, etc.) are evaluated using the test sample set. The partition of the batch, followed by training and evaluation of the model parameters is performed in several iterations. Then, the model parameters are averaged; and if the characteristics are good, the classifier is trained on the full batch.

For comparative analysis of data sets from different subjects, it is preferable to use data sets from subjects living in the same territory. For example, the similarity in the place of residence (a certain territory), type of settlement (city, town, village), nationality, social class is important to minimize the systematic error in determining the abundance of bacterial taxa in the gastrointestinal tract of subjects. To improve the most generalized results, it is also preferable that the cohort is balanced in terms of gender (includes equal proportions of participants of both sexes) and age (different age groups are adequately represented).

**Step 150:** classifying at least one user for whom recommendations for dietary interventions are further generated as a responder or a non-responder based on the algorithm for predicting the degree of individual response, and generating a recommendation for the dietary intervention if the user has been classified as a responder to it.

The following embodiments of the method and the system are given for the purpose of disclosing the characteristics of this invention and should not be regarded as limiting the scope of the invention in any way.

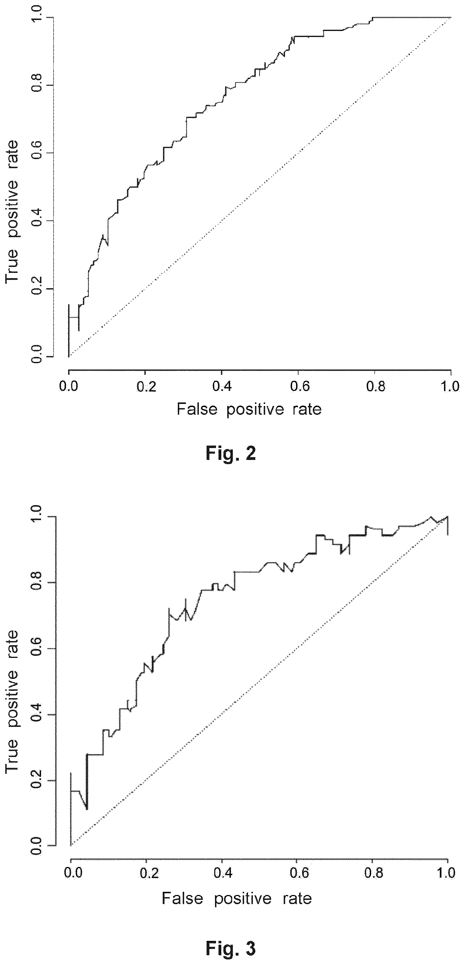

In one embodiment of the invention, data from a non-controlled study of the increase in the intake of fiber-rich products (Klimenko N. S., et al., Nutrients. 2018 May 8;10(5)) were used as data. The study involved 215 people, for two weeks they were on a diet rich in fibers. Microbiota (faeces) samples were collected from the participants before and after the dietary intervention; the samples were analyzed using 16S rRNA sequencing. To divide the subjects into the groups with high or low degree of response to the administration of products, the degree of change of the microbiota composition was used with the Generalized Unifrac metric of β-diversity. The division into two clusters was carried out by applying the k-means method to the distance vector. Taxa significantly differing in the level between the groups with strong and weak reactions of the microbiota to the intervention were searched using the MaAsLin algorithm. Before the diet the group with a high degree of response to the dietary intervention was characterized by a lower relative abundance of the types *Actinobacteria* (order *Coriobacteriales*), *Firmicutes* (orders *Bacillales, Erysipelotrichales* and *Clostridiales), Proteobacteria* (order *Enterobacteriales*) and *Verrucomicrobia* (order *Verrucomicrobiales*), while the abundance of *Bacteroidales* and *Sphingomonadales* was higher than in the group that reacted to the intervention weaker. Functional analysis showed that the group that reacted to the intervention stronger is characterized by a higher abundance of metabolic modules and pathways typical of Gram-negative bacteria: lipopolysaccharide biosynthesis (md:M00060) and NADH:quinone oxidoreductase (md:M00144). The level of the pathway associated with the degradation of carbohydrate mucosa components was higher as well - "Other glycan degradation pathway" (ko00511). The classifier for determining the degree of response based on the microbiota composition was constructed using the random forest algorithm with the use of all bacterial taxa significantly differing in the abundance between the groups before the diet, as predictors. The classifier was constructed and tested at each taxonomic level. Cross-validation (10 random batches, 70% for training, 30% for testing) was carried out. For each iteration, a ROC curve was constructed and the area under the curve was calculated, as shown in Fig. 2, the prediction accuracy was evaluated based on this area. The area under the curve was 0.78 for this classifier.

In one embodiment of the invention, data from a study of the effect of a fermented milk product enriched with a probiotic [Volokh, Olesya, et al. "Human Gut Microbiome Response Induced by Fermented Dairy Product Intake in Healthy Volunteers". Nutrients 11.3 (2019): 547] were used as data. The study involved 150 people, during a month they added 250 ml of yogurt enriched with the probiotic strain *Bifidobacterium animalis* subsp. *lactis* BB-12 to their diet. Microbiota (faeces) samples were collected from the participants before and after the dietary intervention; the samples were analyzed using 16S rRNA sequencing. To divide the subjects into the groups with high or low degree of response to the administration of products, the degree of change of the microbiota composition was used with the Bray-Curtis metric of β-diversity, applied to the abundance of the group of lactose-fermenting bacteria. The division into two clusters was carried out by applying the k-means method to the distance vector. Taxa significantly differing in the relative abundance between the groups with strong and weak reactions to the intervention were searched using the metagenomeSeq algorithm. The group with a high degree of response to the dietary intervention was characterized by a lower abundance of the types *Euryarcheota* (order *Methanobacteria*) and *Firmicutes* (orders *Acidaminococcales, Clostridiales*). Among the function modules, this group had a higher level of Folate biosynthesis (ko00790) compared to the group that reacted to the dietary intervention weaker. The classifier for determining the degree of response based on the microbiota composition was constructed using the random forest algorithm with the use of all bacterial taxa significantly differing between the groups before the diet, as predictors. The classifier was constructed and tested at each taxonomic level. Cross-validation (10 random batches, 70% for training, 30% for testing) was carried out. For each iteration, a ROC curve was constructed and the area under the curve was calculated, as shown in Fig. 3, the prediction accuracy was evaluated based on this area. The area under the curve was 0.75 for this classifier.

Although the invention is described in reference to the embodiments disclosed, it should be clear to a person skilled in the art that the particular cases closely described are given only to illustrate this invention and should not be regarded as limiting the scope of the invention in any way. It should be clear that it is possible to implement various modifications without departing from the spirit of this invention.

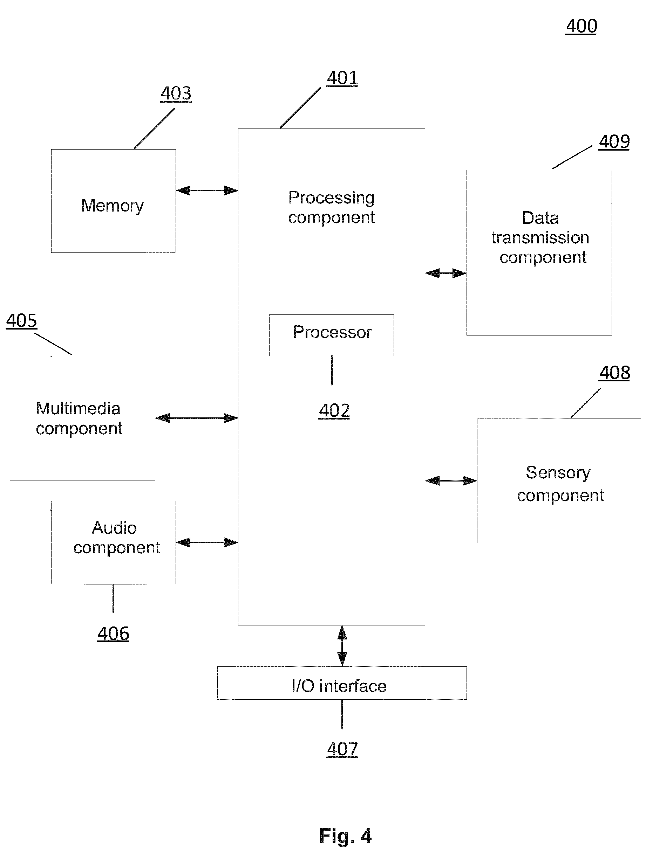

With reference to Fig. 4, the present disclosure can be implemented as a computing system **400** which includes one or more of the following components:
- a processing component **401** including at least one processor **402,**
- a memory **403,**
- a multimedia component **405,**
- an audio component **406,**
- an input/output interface (I/O) **407,**
- a sensory component **408,**
- a data transmission component 409.

The processing component **401** mainly controls all operations of the system **400,** for example, generates a set of hashtags based on the content for the user, as well as controls the display, phone call, data transmission, camera operation and recording operation of the user's mobile communication device. The processing component **401** can include one or more processors **402** executing commands for completion of all or some of the stages from the above-mentioned methods. Moreover, the processing component **401** can include one or more modules for convenient interaction between other processing components **401** and other modules. For example, the processing component **401** can include a multimedia module for convenient easier interaction between the multimedia component **405** and the processing component 401.

The memory **403** is configured to store various types of data to support operation of the system **400,** for example, a database with user profiles. Examples of such data include commands from any application or method, contact details, address book data, messages, images, videos, etc., and they all work on the system 400. The memory **403** can be implemented in the form of any type of volatile memory, non-volatile memory or a combination thereof, for example, static random-access memory (SRAM), electrically erasable programmable read-only memory (EEPROM), erasable programmable read-only memory (EPROM), programmable read-only memory (PROM), read-only memory (ROM), magnetic memory, flash memory, magnetic disk, optical disk, etc., but not limited to the above-mentioned.

The multimedia component **405** includes a screen providing an output interface between the system **400,** which can be installed on user's mobile communication device and by the user. In some embodiments, the screen can be a liquid crystal display (LCD) or a touch panel (TP). If the screen includes a touch panel, the screen can be implemented as a touch screen to receive input from the user. The touch panel includes one or more touch sensors detecting gestures, touching and sliding of the touch panel. The sensor can sense touching borders or flick gestures, as well as determine the duration of time and pressure associated with the touching and sliding modes. In some embodiments, the multimedia component **405** includes one front-facing camera and/or one rear-facing camera. When the system **400** is in an operating mode, for example, in a photo mode or a video mode, the front-facing camera and/or the rear-facing camera can receive multimedia data externally. Each front-facing camera and rear-facing camera can be one fixed lens optical system or have a focal distance or an optical zoom.

The audio component **406** is configured to output and/or input audio signals. For example, the audio component **406** includes one microphone (MIC) that is configured to receive external audio signals when the system **400** is in an operating mode, for example, in a call mode, a record mode and a voice recognition mode. The audio signal received can further be stored in the memory **403** or can be sent to the data transmission component **409**. In some embodiments, the audio component **406** also includes one loudspeaker configured to output audio signals.

The input/output interface (I/O) **407** provides an interface between the processing component **401** and any peripheral interface module. The above-mentioned peripheral interface module can be a keyboard, a steering wheel, a button, etc. These buttons can include a start button, a volume button, an initiate button and a lock button, but not limited to the above-mentioned.

The sensory component **408** may include one or more sensors and is configured to provide various aspects of condition assessment of the system **400**. For example, the sensory component **408** can detect on/off conditions of the system **400,** the relative position of its components, such as the display and the keypad, a single component of the system **400,** the presence or absence of contact between the user and the system **400,** as well as the orientation or acceleration/deceleration and temperature change of the system 400. The sensory component **408** may include a non-contact sensor configured to detect the presence of an object nearby when there is no physical contact. The sensory component **408** may include an optical sensor (for example, a CMOS or a CCD image sensor) configured to be used in the visualization of the application. In some embodiments, the sensory component **408** may include an acceleration sensor, a gyro sensor, a magnetic sensor, a pressure sensor or a temperature sensor.

The data transmission component **409** is configured to facilitate wired or wireless communication between the system **400** and other devices. The system **400** can access a wireless network based on communication standards, such as Wi-Fi, 2G, 3G, 5G or combinations thereof. In an exemplary embodiment, the data transmission component **409** receives a broadcast signal or a broadcast, information related to them from an external broadcast control system through a broadcast channel. In one embodiment, the data transmission component **409** may include a near field communication (NFC) module to facilitate near field communication. For example, the NFC module can be based on radio frequency identification (RFID) technology, infrared data association (IrDA) technology, ultra-wideband (UWB) technology, Bluetooth (BT) technology, etc.

In an exemplary embodiment, the system **400** can be implemented by means of one or more application-specific integrated circuits (ASIC), a digital signal processor (DSP), digital signal processing devices (DSPDs), a programmable logic device (PLD), a field-programmed gate array (FPGA), a controller, a microcontroller, a microprocessor or other electronic components and can be configured to implement the method of generating personalized recommendations for diet based on the analysis of the microbiota composition.

In an exemplary embodiment, a non-volatile computer-readable medium may include prescribed instructions as well, for example, the memory **403** includes instructions, where these instructions are executed by the processor **401** of the system **400** for implementing the above methods of generating personalized recommendations for diet based on the analysis of the microbiota composition. For example, the non-volatile computer-readable medium can be read-only memory (ROM), random-access memory (RAM), a compact disk, a magnetic tape, floppy disks, optical storage devices, etc.

The computing system **400** can include a display interface that transmits graphics, text and other data from the communication infrastructure (or from a frame buffer, not shown) to display on the multimedia component 405. The computing system **400** additionally includes input devices or peripheral devices. The peripheral devices can include one or more devices to interact with the user's mobile communication device, such as a keyboard, a microphone, a wearable device, a camera, one or more audio speakers, and other sensors. The peripheral devices can be external or internal to the user's mobile communication device. The touch screen can usually display graphics and text and also provides a user interface (for example, but not limited to a graphical user interface (GUI)) through which the user can interact with the user's mobile communication device, for example, access and interact with applications running on this device.

The elements of the claimed invention are in functional interrelationships, and their multiple use leads to the creation of a new and unique technical solution. Thus, all the units are functionally related.

All the units used in the system can be implemented using electronic components used to create digital integrated circuits, that is obvious for a person skilled in the art. Chips, the logic of which is specified during manufacturing, or field-programmable gate arrays (FPGAs), the logic of which is specified by programming, can also be used without limitation. For programming, programmers and debugging environments are used that allow to set the desired structure of a digital device in the form of an electrical schematic diagram or a program in special hardware description languages: Verilog, VHDL, AHDL and others. An alternative to FPGAs can be programmable logic controllers (PLC), master slice arrays (MSA), which require a factory production process for programming; ASIC, i.e. application-specific custom-designed large-scale integrated circuits (LSIC), which are much more expensive for small-batch and individual production.

Usually, a FPGA chip itself consists of the following components:
- configurable logic units that implement the required logical function;
- programmable electronic connections between configurable logic units;
- programmable input/output units that provide communication between the external output of the chip and the internal logic.

The units can also be implemented using read-only memory.

Thus, implementation of all used units is achieved by standard means based on the classical principles of implementation of the fundamentals of computer engineering.

As will be understood by a person skilled in the art, the aspects of the present disclosure can be implemented in the form of a system, a method or a computer program product. Accordingly, various aspects of the present disclosure can be implemented solely as hardware, as software (including application software, etc.) or as an embodiment combining software and hardware aspects, which in general may be referred to as "the module", "the system" or "the architecture". Moreover, aspects of the present disclosure can take the form of a computer program product implemented on one or more computer-readable media having a computer-readable program code that is implemented on them.

Any combination of one or more machine-readable media can also be used. A machine-readable storage medium can be, without limitation, an electronic, magnetic, optical, electromagnetic, infrared or semiconductor system, a device, an apparatus or any suitable combination thereof. More specifically, examples (non-exhaustive list) of machine-readable storage media include: an electrical connection using one or more wires, a portable computer floppy disk; a hard disk, random-access memory (RAM), read-only memory (ROM), erasable programmable read-only memory (EPROM or flash memory), a fiber optic connection, compact disc read-only memory (CD-ROM), an optical storage device, a magnetic storage device or any combination thereof. In the context of this description, a machine-readable storage medium can be any flexible data medium that can include or store a program for use by the system, device, apparatus, or in conjunction therewith.

The program code embedded in the machine-readable medium can be transmitted by any medium, including, without limitation, wireless, wired, fiber optic, infrared and any other suitable network or a combination thereof.

The computer program code for performing operations for the stages of the present disclosure can be written in any programming language or a combination of programming languages, including an object-oriented programming language such as Java, Smalltalk, C++, etc., and conventional procedural programming languages such as the programming language "C" or similar programming languages. The program code can be executed on the user's computer in full, in part, or as a separate software package, partly on the user's computer and partly on the remote computer, or completely on the remote computer. In the latter case, the remote computer can be connected to the user's computer via any type of network, including a local area network (LAN), a wide area network (WAN) or a connection to an external computer (for example, over the Internet using Internet service providers).

The aspects of the present disclosure have been closely described with reference to flow charts, circuit diagrams and/or diagrams of methods, devices (systems) and computer program products in accordance with embodiments of the present disclosure. It should be noted that each component of the flow chart and/or diagrams, as well as combinations of components of the flow chart and/or diagrams can be implemented with computer program commands. These computer program commands can be provided to a general-purpose computer processor, a special-purpose computer processor or another data processing device to create a procedure in such a way that the commands executed by the computer processor or other programmable data processing device create the means for implementing the functions/actions specified in a component or components of the flow chart and/or the diagram.

These computer program commands can also be stored on a machine-readable medium that can control a computer other than a programmable data processing device or other than devices that operate in a specific manner in such a way that the commands stored on the machine-readable medium create a device including commands that perform the functions/actions specified in a component of the flow chart and/or the diagram.

## Claims

1. A computer-implemented method of generating a recommendation for a user for a dietary intervention, performed on at least one processor, comprising the following stages:
• obtaining a set consisting of at least one proposed dietary intervention for the user and a user's data including at least data on a composition of intestinal microbiota of the user before the proposed dietary intervention;
• retrieving data sets on changes in a composition of intestinal microbiota of subjects after the proposed dietary intervention by means of a processor from at least one database of dietary interventions, while selecting data sets of subjects residing in the same territory as the user;
• assigning each selected subject to a group with a high degree of response (responder) to the proposed dietary intervention or to a group with a low degree of response (non-responder) by using a value of changes in the composition of intestinal microbiota of the subject after the dietary intervention as a measure of the degree of response;
• generating an algorithm for predicting the degree of individual response to the proposed dietary intervention based on retrieved changes in the composition of intestinal microbiota and the response group to the proposed dietary intervention of the subjects, which classifies a user as a responder or a non-responder, from the earlier obtained data on the composition of the intestinal microbiota and the group of response to the proposed dietary intervention;
• classifying the user as a responder or a non-responder based on the user's data the algorithm for predicting the degree of individual response, and
• generating a recommendation for the user for the dietary intervention based on the results of the classification.

2. The method of claim 1, wherein the degree of response to the intervention is assessed additionally based on biochemical tests and/or questionnaires on gastrointestinal symptoms and/or defecation frequency and/or well-being.

3. The method of claim 2, wherein the biochemical test is an analysis of the level of triglycerides and other indicators in the blood and/or a analysis of faeces and/or a microbiological analysis of faeces.

4. The method of claim 1, wherein a random forest algorithm, a support vector machine or an artificial neural network is used as an algorithm for predicting the degree of individual response.

5. The method of claim 1, wherein subjects are assigned to the group of responders to the dietary intervention, for whom the value of changes in the composition of intestinal microbiota after the proposed dietary intervention exceeded a threshold value, or to the group of non-responders for whom this value did not exceed the threshold value.

6. The method of claim 1, wherein data set from users and/or subjects are obtained by analyzing microbiota samples obtained from the users/subjects and containing microorganisms present in their gastrointestinal tract.

7. The method of claim 6, wherein the obtained microbiota samples are samples of faeces.

8. The method of claim 6, wherein data set from the users and/or subjects are obtained by sequencing libraries prepared from DNAs extracted from the microbiota sample with the use of enzymatic lysis and/or mechanical lysis and/or washout.

9. The method of claim 6, further comprising is DNA sequencing the whole or partial sequence of 16S rRNA genes or metagenomic whole genome sequencing.

10. The method of claim 1, wherein the data on the composition of intestinal microbiota values comprises data on an abundance of microorganisms present in the a gastrointestinal tract including relative abundance microbial taxa, α-diversity, β-diversity and/or abundance of preset taxa.

11. The method of claim 1, wherein the dietary intervention is a long-term administration of a product or a variety of products rich in a certain class of dietary fibers, prebiotics, probiotics or a food product enriched with prebiotics or probiotics.

12. The method of claim 1, wherein the dietary intervention is fasting therapy.

13. The method of claim 1, wherein the recommendation is to exclude a certain food product, probiotic or prebiotic from the diet based on that the user has been assigned to the group of non-responders to proposed dietary intervention.

14. The method of claim 1, wherein the recommendation is generated in reference to several proposed dietary interventions.

15. A computer system for generating a recommendation for dietary interventions for a user, comprising at least one processor and at least one memory storage comprising a recorded program for implementing the stages of the method of claim 1 and connected to the processor.
